(19) Europäisches Patentamt
European Patent Office
Office européen des brevets

(11) **EP 2 308 591 B1**

(12) **EUROPEAN PATENT SPECIFICATION**

(45) Date of publication and mention
of the grant of the patent:
**25.07.2018 Bulletin 2018/30**

(51) Int Cl.:
*B01J 20/20* (2006.01)   *B01J 20/28* (2006.01)
*A61K 8/97* (2017.01)    *A61K 33/44* (2006.01)
*A61P 13/12* (2006.01)   *A61Q 19/10* (2006.01)
*A61K 8/19* (2006.01)    *A61K 8/25* (2006.01)
*A61Q 19/00* (2006.01)   *A23L 33/105* (2016.01)
*C01B 32/30* (2017.01)   *C01B 32/324* (2017.01)
*C01B 32/336* (2017.01)

(21) Application number: **09803027.3**

(22) Date of filing: **30.07.2009**

(86) International application number:
**PCT/JP2009/063599**

(87) International publication number:
**WO 2010/013785 (04.02.2010 Gazette 2010/05)**

(54) **USE OF A POROUS ACTIVE CARBON MATERIAL AS ADSORBENT, REMEDY FOR KIDNEY DISEASE AND FUNCTIONAL FOOD**

VERWENDUNG EINES PORÖSES AKTIVKOHLEMATERIAL ALS ADSORBENT, MEDIKAMENT FÜR NIERENERKRANKUNGEN UND FUNKTIONSNAHRUNG

UTILISATION D'UN MATÉRIAU DE CHARBON ACTIF POREUX COMME ADSORBANT, REMÈDE POUR UNE MALADIE RÉNALE ET ALIMENT FONCTIONNEL

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **31.07.2008   JP 2008197513**
**02.10.2008   JP 2008257133**
**08.07.2009   JP 2009161672**

(43) Date of publication of application:
**13.04.2011 Bulletin 2011/15**

(73) Proprietor: **Sony Corporation**
**Tokyo 108-0075 (JP)**

(72) Inventors:
• **TABATA, Seiichiro**
**Tokyo 108-0075 (JP)**
• **IIDA, Hironori**
**Tokyo 108-0075 (JP)**
• **HORIE, Takeshi**
**Tokyo 108-0075 (JP)**
• **YAMADA, Shinichiro**
**Tokyo 108-0075 (JP)**
• **NOGUCHI, Tsutomu**
**Tokyo 108-0075 (JP)**

(74) Representative: **Müller Hoffmann & Partner**
**Patentanwälte mbB**
**St.-Martin-Strasse 58**
**81541 München (DE)**

(56) References cited:
EP-A1- 2 060 535        EP-A1- 2 324 854
EP-A1- 2 330 078        WO-A1-96/27911
JP-A- 2005 296 933      JP-A- 2005 305 406
JP-A- 2006 143 736      JP-A- 2006 256 882
JP-A- 2008 024 611      JP-B2- 3 565 994

• Miguel A. Schettino ET AL: "PREPARAÇÃO E CARACTERIZAÇÃO DE CARVÃO ATIVADO QUIMICAMENTE A PARTIR DA CASCA DE ARROZ", Quim. Nova, 29 August 2007 (2007-08-29), pages 1663-1668, XP55010660, Brazil Retrieved from the Internet: URL:http://www.scielo.br/pdf/qn/v30n7/30.p df [retrieved on 2011-10-27]

EP 2 308 591 B1

## Description

Technical Field

The present invention relates to the use of an adsorbent, which includes a porous carbon material produced from a plant-derived material.

Background Art

Most of unused portions of plants such as vegetables and cereals are discarded, and effective utilization of these unused portions is keenly demanded for preservation and/or improvement of global environment. Examples of the effective utilization of the unused portions include a carbonizing treatment. Use of a carbon material, produced by a carbonizing treatment of such a plant-derived material as above-mentioned, as a negative electrode active material in lithium ion secondary batteries has also been investigated (see, for example, Japanese Patent Nos. 3565994 and 3719790, and PCT Patent Publication No. WO96/27911 pamphlet).

In the body of a patient of a hepatic or renal disease, various toxic substances are liable to be produced and accumulated due to organopathy, thereby causing uremia or disorder of consciousness or the like. In addition, for patients of a renal disease (chronic renal failure), removal of toxic substances by hemodialysis is performed. However, hemodialysis not only needs a special apparatus and a technician but also imposes considerable physical and mental burdens on the patient. As means for delaying the timing of adoption of the hemodialysis, oral activated-carbon adsorbents such as Kremezin which are high in stability and safety for living bodies are drawing attention (see Japanese Patent Publication No. Sho 62-11611). This approach is to remove uremic metabolites and/or uremic toxin in digestive apparatuses by the activated-carbon adsorbent, so as to restrain the progress of chronic renal failure. Besides, antiobestic drugs, antidiabetic drugs, inflammatory bowel disease drugs and purine body adsorbents which are based on the use of activated carbon have also been proposed. Thus, applications as well as research and development of activated carbon in medical fields have also been being carried out widely.

Cleansing agents (detergents, and cosmetic or detergent adsorbents) contain higher fatty acid salts and/or various surfactants as main ingredients, and enhancement of the detergency thereof relies on increases in the contents of these ingredients. However, the cleaning action of the higher fatty acid salt or surfactant is realized through emulsification or solubilization of sebum on the skin, and excessive cleaning as a result of increases in the contents of these ingredients would cause rough dry skin and/or a tight feeling of skin. In recent years, cleansing agents free of a feeling of excessive cleansing of skin after use thereof have been developed by admixing the higher fatty acid salt with charcoal (carbon) so as to enhance the detergency without increasing the loadings of higher fatty acid salts and/or various surfactants (see, for example, Japanese Patent Laid-open No. 2002-167325). At present, a multiplicity of cleansing stuffs based on loading of charcoal, activated carbon or medicinal carbon are commercially available, and these commercial products include those which are stated to have a body order removing effect (deodorant effect) or antibacterial effect in addition to a high detergent effect and those which are said to have the merit of high safety (see, for example, Japanese Patent Laid-open Nos. Hei 9-111296 and 2000-53558) .

Prior Art Documents

Patent Documents

Patent Document 1: Japanese Patent No. 3565994
Patent Document 2: Japanese Patent No. 3719790
Patent Document 3: PCT Patent Publication No. WO96/27911 pamphlet
Patent Document 4: Japanese Patent Publication No. Sho 62-11611
Patent Document 5: Japanese Patent Laid-open No. 2002-167325
Patent Document 6: Japanese Patent Laid-open No. Hei 9-111296
Patent Document 7: Japanese Patent Laid-open No. 2000-53558

EP 2 060 535 A1, which is prior art according to Art. 54(3) EPC, discloses a porous carbon material obtainable from a plant-derived material having a silicon content of at least 5 wt% as a raw material and having a value of specific surface area of at least 10 m2/g as measured by the nitrogen BET method, a silicon content of at most 1 wt% and a pore volume of at least 0.1 cm3/g as measured by the BJH method and MP method.

Schettino M. A. et al. (Quim. Nova, Vol. 30, No. 7, 1663 - 1668, 2007) relates to the preparation and characterization of chemically activated carbon from rice hulls.

EP 2 324 854 A1, which is prior art according to Art. 54(3) EPC, provides a drug sustained-release agent including a carbon material (porous carbon material) which has an inverse opal structure. The drug sustained-release agent includes a porous carbon material which has spherical pores having an average diameter of 1 x 109 to 1 x 105 m and arrayed three-dimensionally and which has a surface area of 3 x 102 m2/g.

EP 2 330 078 A1 which is prior art according to Art. 54(3) EPC, relates to a porous carbon material composite formed of a porous carbon material and a functional material and equipped with high functionality. A porous carbon material composite is formed of (A) a porous carbon material obtainable from a plant-derived material having a silicon (Si) content of 5 wt% or higher as a raw material, said porous carbon material having a silicon content of 1 wt% or lower, and (B) a functional material adhered to the porous carbon material, and has a specific surface area of 10 m2/g or greater as determined by the nitrogen BET method and a pore volume of 0.1 cm3/g or greater as determined by the BJH method and MP method.

Summary of the Invention

However, technologies for carbonizing treatment of plant-derived materials are not yet sufficient, and the carbon materials produced are desired to be enhanced more in functionality. In addition, there is a keen request for development of porous carbon materials for oral adsorbents to be used in treatment of renal and hepatic diseases, or for development of porous carbon materials which are aimed at adsorption of viruses or proteins having bad influences on human bodies or are to be used as medical adsorbents being more excellent in adsorption performance. Specifically, materials which exhibit a great adsorption amount for toxic substances when used in a smaller dose are keenly desired. Where such a material is used, it is possible, by more reducing the amount of the material orally administered at a time, to alleviate the burden on the patient.

Besides, the existing cleansing agents contain charcoal (carbon) simply blended therein, and they cannot be said to be sufficient in cleaning effect or skin-conditioning effect. The charcoal (carbon) component in the existing cleansing agents is merely showing an auxiliary role in chemosynthetic detergents.

Accordingly, it is an object of the present invention to provide an use of an adsorbent, which comprises a porous carbon material having an excellent adsorption performance. This is achieved by the subject-matter of claim 1.

Adsorbents for attaining the above-mentioned object, include a porous carbon material which is produced from a plant-derived material having a silicon content of not less than 5 wt.% and which has a value of specific surface area determined by the nitrogen BET method of not less than 10 m$^2$/g, a silicon content of not more than 1 wt.%, and pore volumes determined by the BJH method and the MP method of not less than 0.1 cm$^3$/g.

Here, the hydrophobic molecules can be defined as sebum present on human skin surfaces and oils and fats or dirt which may be deposited on skin or clothing. Specific examples of the hydrophobic molecules include: oils and fats or sebum containing a fatty acid glycerin ester; lipids such as simple lipids, compound lipids, and induced lipids; and organic acids constituting wax, especially those oily components present on human skin surfaces in general (fatty acids in a wide sense). Besides, the examples also include fatty acids in a narrow sense which have a straight chain or branched chain or have a cyclic structure, such as saturated fatty acids and unsaturated fatty acids. Specific examples of the fatty acids include oleic acid, stearic acid, myristic acid, squalene (oil or fat belonging to terpenoid), cholesterol (biosynthesized from squalene), and monoglycerin stearate. Besides, examples of the coloring matter include authorized dyes (coal-tar dyes), such as Lithol Rubine BCA (Red No. 202), in general, that is, Amaranth (Red No. 2), New Coccine (Red No. 102), Lithol Rubine B (Red No. 201), Lithol Red CA (Red No. 206), Rhodamine B (Red No. 231), Deep Maroon (Red No. 220), Fast Acid Magenta (Red No. 227), Violamine R (Red No. 401), Scarlet Red NF (Red No. 501), Fast Red S (Red No. 506), Dibromofluoresceine (Orange No. 201), Diodofluoresceine (Orange No. 206), Hansa Orange (Orange No. 401), Tartrazine (Yellow No. 4), Fluoresceine (Yellow No. 201), Benzidine Yellow G (Yellow No. 205), Hansa Yellow (Yellow No. 401), Metanil Yellow (Yellow No. 406), Fast Green FCF (Green No. 3), Arizarine Cyanine Green F (Green No. 201), Naphthol Green B (Green No. 401), Brilliant Blue FCF (Blue No. 1), Indigo (Blue No. 201), Sudan Blue B (Blue No. 403), Resorcine Brown (Brown No. 201), Alizurine Purple SS (Violet No. 201), and Naphthol Blue Black (Black No. 401).

Incidentally, the adsorbent that adsorbs indole is applicable to drug for renal diseases such as uremia. The adsorbent that adsorbs uric acid is applicable to drug for renal diseases such as uremia and to drug for hyperuricemia. The adsorbent that adsorbs adenosine is applicable to a purine body adsorbent. The adsorbent that adsorbs α-amylase is applicable to a model for examination of adsorption characteristics of protein such as inflammatory cytokine which causes Crohn's disease or the like (simulated inflammatory cytokine). The adsorbent that adsorbs 3-methylindole is applicable to drug for renal diseases such as uremia. The adsorbent that adsorbs trpyptophan is applicable to drug for renal disease such as uremia. The adsorbent that adsorbs indicant is applicable to drug for renal diseases such as uremia. The adsorbent that adsorbs theophylline is applicable to an antidotal adsorbent for drug poisoning (theophylline poisoning). The adsorbent that adsorbs inosine 5-monophosphate disodium salt is applicable to a purine body adsorbent.

The adsorbent that adsorbs adenosine 5-triphosphate disodium salt is applicable to a purine body adsorbent. The adsorbent that adsorbs a fatty acid, the adsorbent that adsorbs a coloring matter, are for removing dirt components of sweat, oils and fats, a red cosmetic for the lips, etc. and may have a skin-conditioning function.

A renal disease drug includes a porous carbon material which is produced from a plant-derived material having a silicon content of not less than 5 wt.% and which has a value of specific surface area determined by the nitrogen BET method of not less than 10 m$^2$/g, a silicon content of not more than 1 wt.%, and pore volumes determined by the BJH method and the MP method of not less than 0.1 cm$^3$/g.

A functional food includes a porous carbon material which is produced from a plant-derived material having a silicon content of not less than 5 wt.% and which has a value of specific surface area determined by the nitrogen BET method of not less than 10 m$^2$/g, a silicon content of not more than 1 wt.%, and pore volumes determined by the BJH method and the MP method of not less than 0.1 cm$^3$/g. Incidentally, the functional food according to the invention may contain other ingredients than the porous carbon material; examples of the other ingredients include excipient, binder, disintegrator, lubricant, diluent, flavoring agent (corrigent), preservative, stabilizer, colorant, perfume, vitamins, color fixative, brightener, sweetening agent, bitterness agent, sour agent, taste enhancer, fermentative seasoning, antioxidant, enzyme, yeast extract, and nutritional supplement. Besides, examples of the form of the functional food include powder, solid, tablets, particles, granules, capsules, cream, sol, gel, and colloid.

In the porous carbon material in each of the adsorbents used according to the embodiments of the present invention, may hereinafter be generically referred to as "the adsorbent or the like used according to the present invention"), it is preferable, though not limitative, that the content of magnesium (Mg) is 0.01 to 3 wt.%, the content of potassium (K) is 0.01 to 3 wt.%, and the content of calcium (Ca) is 0.05 to 3 wt.%.

In addition, the porous carbon material in the adsorbent used according to the present invention can be obtained by carbonizing the plant-derived material at 800 to 1400°C and then treating the carbonization product with an acid or alkali. Incidentally, such a production method will be referred to as "first production method." Or, alternatively, the porous carbon material can be obtained by subjecting the plant-derived material to a pre-carbonizing treatment (described later), then treating the resulting product with an acid or alkali, and carbonizing the thus treated product at 800 to 1400°C. Incidentally, such a production method will be referred to as "second production method." Here, the term "carbonization" generally means conversion of an organic material (in the present invention, the plant-derived material) to a carbon material by a heat treatment (see, for example, JIS M0104-1984). Incidentally, examples of the atmosphere for the carbonization include an atmosphere shut out from oxygen; more specific examples include a vacuum atmosphere, an inert gas atmosphere such as nitrogen gas or argon gas, and an atmosphere for baking the plant-derived material in (or as if in) a covered casserole. The rate of temperature rise until a carbonizing temperature is reached, in such an atmosphere as just-mentioned, may be not less than 1°C/minute, preferably not less than 3°C/minute, and more preferably not less than 5°C/minute. The upper limit for carbonization time may be 10 hours, preferably 7 hours, and more preferably 5 hours, the values being not limitative. The lower limit for the carbonization time may be such that the plant-derived material is carbonized assuredly in the limited time. Besides, the plant-derived material may be pulverized to a desired grain size, as required, and may further be subjected to classification. In addition, the plant-derived material may be preliminarily cleaned.

In the first production method or the second production method for the porous carbon material in the adsorbent or the like used according to the present invention, the porous carbon material obtained may be subjected to an activating treatment, whereby it is possible to increase micropores (described later) smaller than 2 nm in pore diameter. Examples of the activating methods include gas activating methods and chemical agent activating methods. Here, the gas activating methods are methods in which oxygen, steam (water vapor), carbon dioxide gas, air or the like is used as an activating agent, and the porous carbon material is heated in such a gas atmosphere at 700 to 1000°C for a period of time ranging from several tens of minutes to several hours, thereby developing a microstructure by utilizing volatile components or carbon molecules in the porous carbon material. Incidentally, the heating temperature may be appropriately selected based on the kind of the plant-derived material, the kind and concentration of the gas used, etc., and is preferably 800 to 950°C. The chemical agent activating methods are methods in which the porous carbon material is activated by use of zinc chloride, iron chloride, calcium phosphate, calcium hydroxide, magnesium carbonate, potassium carbonate, sulfuric acid or the like, in place of the oxygen or steam used in the gas activating methods, then the porous carbon material is washed in hydrochloric acid, and pH adjustment with an aqueous alkaline solution is conducted, followed by drying. Besides, preferably, silicon components in the plant-derived material after the carbonization are removed by a treatment with an acid or an alkali. Here, examples of the silicon components include silicon oxides such as silicon dioxide, silicon oxide, and silicon oxide salts.

In addition, in the first production method for the porous carbon material in the adsorbent used according to the present invention, the plant-derived material may, before carbonization, be subjected to a heating treatment (pre-carbonizing treatment) in a condition shut out from oxygen at a temperature lower than the carbonizing treatment (for example, at a temperature of 400 to 700°C), though depending on the plant-derived material used. By this heating treatment or in the second production method, tar components which would be produced in the carbonization process can be extracted, resulting in that the tar components which would be produced in the carbonization process can be reduced in amount or be removed. Incidentally, the condition shut out from oxygen can be achieved by use of an inert gas atmosphere such as nitrogen gas or argon gas, or by use of a vacuum atmosphere, or by baking the plant-derived material in (or as if in) a covered casserole. Besides, in the first production method or the second production method, the plant-derived material may be immersed in an alcohol (for example, methyl alcohol, ethyl alcohol, or isopropyl alcohol) for the purpose of reducing the amounts of mineral components and water (moisture) contained in the plant-derived material and/or for the purpose of preventing the generation of foreign odors in the carbonization process, though depending on the plant-derived material used. Incidentally, the first production method may be followed by the pre-carbonizing treatment. Examples of material for which a heating treatment in an inert gas is preferred include plants which generate large amounts of wood vinegar (tar or light oil). On the other hand, examples of material for which a pretreatment with an alcohol is preferred include marine algae containing iodine and/or various minerals in large amounts.

A surface of the porous carbon material in the adsorbent used according to the present invention may be subjected to a chemical treatment or molecular modification. Examples of the chemical treatment include a treatment in which carboxyl groups are produced on the surface by treatment with nitric acid. Besides, a treatment similar to the activating treatment by use of steam, oxygen, alkali or the like may be conducted, so as to produce various functional groups such as hydroxyl group, carboxyl group, ketone group, ester group, etc. on the surface of the porous carbon material. Further, the porous carbon material may also be brought into chemical reaction with a chemical species or protein having hydroxyl groups, carboxyl groups, amino groups or the like capable of reaction with the porous carbon material, whereby molecular modification can be achieved.

In the porous carbon material in the adsorbent used according to the present invention inclusive of the above-described various preferable modes and configurations (such adsorbent or the like may hereinafter be generically referred to simply as "the present invention"), examples of the plant-derived material include, but are not limited to, chaff and straws of rice, barley, common wheat, rye, Japanese millet, foxtail millet, etc. as well as common reed and *kukiwakame* seaweed. Further examples of the plant-derived material include vascular plant (Tracheophyta) vegetating on land, Pteridophyta, Bryophyta, algae, seaweed, etc. Incidentally, these materials may be used either singly or in combination of some of them, as raw material. Besides, the shape and form of the plant-derived material are not particularly limited; for example, the chaff or straws may be used per se, or dried products thereof may be used. Furthermore, the materials obtained by subjecting the plant-derived material to various treatments such as fermentation, roasting, extraction, etc. in food and drink processing in the production of beer, foreign liquors or the like can also be used. Especially, from the viewpoint of resource recovery from industrial wastes, it is preferable to use straws or chaff obtained after such processing as threshing. Such straws and chaff after processing are available from Agricultural Cooperatives, alcoholic beverage manufacturers, or food companies in large amounts and easily.

Those materials which are obtained by carbonizing the plant-derived material at 800 to 1400°C and which are not yet treated with an acid or alkali, in the description of the adsorbent used according to the present invention inclusive of the above-mentioned various preferable modes and configurations, will be referred to as "porous carbon material precursor [1]." On the other hand, those materials which are obtained by subjecting the plant-derived material to the pre-carbonizing treatment and which are not yet treated with an acid or alkali will be referred to as "porous carbon material precursor [2]."

The porous carbon material used according to the present invention may contain a nonmetallic element such as phosphorus (P), sulfur (S), etc. or a metallic element such as transition elements. The content of phosphorus (P) may be 0.01 to 3 wt.% and the content of sulfur (S) may be 0.01 to 3 wt.%, for example. Incidentally, though depending on the intended use of the porous carbon material, the contents of these elements and the above-mentioned magnesium (Mg), potassium (K) and calcium (Ca) are more preferable when they are smaller, from the viewpoint of an increase in the value of specific surface area. Naturally, the porous carbon material may contain other elements than the above-mentioned elements, and the ranges of the contents of the above-mentioned various elements can be varied according to the intended use of the porous carbon material.

In the present invention, analysis of the various elements can be carried out by an energy dispersive spectrometry (EDS), for example, using an energy dispersive type X-ray analyzer (e.g., JED-2200F made by JEOL Ltd.). Here, the measuring conditions may, for example, be a scanning voltage of 15 kV and an irradiation current of 13 $\mu$A.

The porous carbon material used according to the present invention has many pores. The pores include "mesopores" having a pore diameter of 2 to 50 nm and "micropores" having a pore diameter of less than 2 nm. Specifically, the mesopores include, for example, many pores having a pore diameter of not more than 20 nm, particularly, many pores having a pore diameter of not more than 10 nm. In addition, the micropores include, for example, many pores with a pore diameter of about 1.9 nm, many pores with a pore diameter of about 1.5 nm, and many pores with a pore diameter of about 0.8 to 1 nm. In the porous carbon material used according to the present invention, the pore volumes determined by the BJH method and the MP methods are each not less than 0.1 $cm^3$/g. Preferably, the pore volume determined by the BJH method is not less than 0.3 $cm^3$/g, and the pore volume determined by the MP method is not less than 0.10 $cm^3$/g. More preferably, the pore volume determined by the BJH method is not less than 0.3 $cm^3$/g, and the pore volume determined by the MP method is not less than 0.15 cm$^3$/g. Further preferably, the pore volume determined by the BJH method is not less than 0.3 cm$^3$/g, and the pore volume determined by the MP method is not less than 0.20 cm$^3$/g. In addition, in the porous carbon material used according to the present invention, the value of specific surface area determined by the nitrogen BET method (the value may hereinafter be referred to simply as "value of specific surface area") is preferably not less than 50 m$^2$/g, more preferably not less than 100 m$^2$/g, for obtaining further excellent functionality.

Or, the porous carbon material used according to the present invention may have a pore size distribution determined by the mercury intrusion method in which a peak is present in the range from $1 \times 10^{-7}$ to $5 \times 10^{-6}$ m and a pore size distribution determined by the BJH method in which a peak is present in the range from 2 nm to 20 nm. In this case, preferably, the pore size distribution determined by the mercury intrusion method has a peak in the range from $2 \times 10^{-7}$ to $2 \times 10^{-6}$ m, and the pore size distribution determined by the BJH method has a peak in the range from 2 to 10 nm.

The nitrogen BET method is a method in which nitrogen as adsorbate molecules is adsorbed onto and desorbed from the adsorbent (here, the porous carbon material) to measure an adsorption isotherm, and the measurement data is analyzed based on a BET formula represented by the formula (1). Based on this method, specific surface area and pore volume and the like can be calculated. Specifically, in the case of calculating the specific surface area by the nitrogen BET method, first, nitrogen as adsorbate molecules is adsorbed onto and desorbed from the adsorbent (porous carbon material), to obtain the adsorption isotherm. Then, from the adsorption isotherm thus obtained, $[p/\{V_a(p_0\text{-}p)\}]$ is calculated based on the formula (1) or on the formula (1') obtained by deformation of the formula (1), and the calculation result is plotted against the equilibrium relative pressure $(p/p_0)$. Next, regarding the plot as a straight line, the inclination $s (= [(C\text{-}1)/(C \cdot V_m)])$ and the intercept $i (= [1/(C \cdot V_m)])$ of the straight line are calculated based on the least squares method. Then, from the inclination s and the intercept $i$ thus obtained, $V_m$ and C are calculated based on the formula (2-1) and the formula (2-2). Further, the specific surface area $a_{sBET}$ is calculated from $V_m$ based on the formula (3) (see the manual for BELSORP-mini and BELSORP analysis software, made by BEL Japan, Inc., pp. 62 to 66). Incidentally, the nitrogen BET method is a measuring method according to the "Measuring method for specific surface area of fine ceramic powders by gas adsorption BET method" defined by JIS R 1626-1996.

$$V_a = (V_m \cdot C \cdot p) / [(p_0 - p)\{1 + (C-1)(p/p_0)\}] \tag{1}$$

$$[p/\{V_a(p_0 - p)\}]$$
$$= [(C-1)/(C \cdot V_m)](p/p_0) + [1/(C \cdot V_m)] \tag{1'}$$

$$V_m = 1/(s+i) \tag{2-1}$$

$$C = (s/i) + 1 \tag{2-2}$$

$$a_{sBET} = (V_m \cdot L \cdot \sigma)/22414 \tag{3}$$

where

$V_a$: adsorption amount
$V_m$: adsorption amount of monomolecular layer
p: pressure of nitrogen at equilibrium
$p_0$: saturated vapor pressure of nitrogen
L: Avogadro's number
$\sigma$: adsorption cross section of nitrogen.

In the case of calculating the pores volume $V_p$ by the nitrogen BET method, for example, linear interpolation is applied to the adsorption data of the adsorption isotherm obtained, and the adsorption amount V at a relative pressure set by a pore volume calculation relative pressure is obtained. From this adsorption volume V, the pore volume $V_p$ can be calculated based on the formula (4) (see the Manual for BELSORP-mini and BELSORP analysis software, made by BEL Japan, Inc., pp. 62 to 65). Incidentally, the pore volume based on the nitrogen BET method may hereinafter be referred to simply as "pore volume").

$$V_p = (V/22414) \times (M_g/\rho_g) \qquad (4)$$

where

V: adsorption amount at relative pressure
$M_g$: molecular weight of nitrogen
$\rho_g$: density of nitrogen.

The pore diameter of mesopores can, for example, be calculated as pore size distribution from the pore volume variation rate relative to the pore diameter, based on the BJH method. The BJH method is a method which is widely used as a pore size distribution analyzing method. In the case of analyzing the pore size distribution based on the BJH method, first, nitrogen as adsorbate molecules is adsorbed onto and desorbed from the adsorbent (porous carbon material), to obtain a desorption isotherm. Next, based on the desorption isotherm thus obtained, the thickness of an adsorbed layer at the time of stepwise adsorption/desorption of adsorbate molecules from the condition where the pores are filled with the adsorbate molecules (e.g., nitrogen) and the inside diameter (twice the core radius) of the pores generated in that instance are obtained, then the pore radius $r_p$ is calculated based on the formula (5), and the pore volume is calculated based on the formula (6). Then, based on the pore radius and the pore volume, the pore volume variation rate ($dV_p/dr_p$) relative to the pore diameter ($2r_p$) is plotted, whereby the pore size distribution curve is obtained (see the Manual for BELSORP-mini and BELSORP analysis software, made by BEL Japan, Inc., pp. 85 to 88).

$$r_p = t + r_k \qquad (5)$$

$$V_{pn} = R_n \cdot dV_n - R_n \cdot dt_n \cdot c \cdot \Sigma A_{pj} \qquad (6)$$

where

$$R_n = r_{pn}^2 / (r_{kn} - 1 + dt_n)^2 \qquad (7)$$

where

$r_p$: pore radius
$r_k$: core radius (inside diameter/2) in the case where an adsorbed layer with a thickness $t$ is adsorbed on the inner wall of pores with a pore radius $r_p$ at that pressure
$V_{pn}$: pore volume when n-th adsorption/desorption of nitrogen is generated
$dV_n$: variation in that instance
$dt_n$: variation of thickness $t_n$ of the adsorbed layer when the n-th adsorption/desorption of nitrogen is generated
$r_{kn}$: core radius in that instance
c: constant
$r_{pn}$: pore diameter when the n-th adsorption/desorption of nitrogen is generated. Besides, $\Sigma A_{pj}$ is the integrated value of the area of wall surfaces of pores from j=1 to j=n-1.

The pore diameter of micropores can, for example, be calculated as pore size distribution from the pore volume variation rate relative to the pore diameter, based on the MP method. In the case of analyzing the pore size distribution by the MP method, first, nitrogen is adsorbed onto the adsorbent (porous carbon material), to obtain an adsorption isotherm. Next, the adsorption isotherm is converted into pore volume relative to the thickness $t$ of the adsorbed layer (plotted against $t$). Then, based on the curvature of the plot (variation of pore volume relative to variation in thickness $t$ of adsorbed layer), a pore size distribution curve can be obtained (see the Manual for BELSORP-mini and BELSORP analysis software, made by BEL Japan, Inc., pp. 72 to 73 and p.82).

The porous carbon material precursors [1] and [2] are treated with an acid or alkali. Specific examples of the treating method include a method in which the porous carbon material precursor [1] or [2] is immersed in an aqueous solution of the acid or alkali, and a method in which the porous carbon material precursor [1] or [2] is reacted with the acid or alkali in a gaseous phase. More specifically, in the case of treatment with an acid, examples of the acid include acidic fluorine compounds such as hydrogen fluoride, hydrofluoric acid, ammonium fluoride, potassium fluoride, sodium fluoride, etc. In the case of using a fluorine compound, it suffices that the amount of the fluorine element is four times the amount of silicon element in the silicon components contained in the porous carbon material precursor [1] or [2], and it is preferable that the concentration of the aqueous solution of the fluorine compound is not less than 10 wt.%. In the case of removing the silicon components (e.g., silicon dioxide) contained in the porous carbon material precursor [1] or [2] by hydrofluoric acid, silicon dioxide is reacted with hydrofluoric acid as represented by the chemical formula (1) or (2), and is removed as hexafluorosilicic acid ($H_2SiF_6$) or silicon tetrafluoride ($SiF_4$), whereby a porous carbon material can be obtained. It suffices that the porous carbon material is then washed and dried.

$$SiO_2 + 6HF \rightarrow H_2SiF_6 + 2H_2O \qquad (1)$$

$$SiO_2 + 4HF \rightarrow SiF_4 + 2H_2O \qquad (2)$$

Besides, in the case of treatment with an alkali (base), for example, sodium hydroxide may be mentioned as the alkali. Where an aqueous solution of an alkali is used, it suffices that the pH of the solution is not less than 11. In the case where the silicon components (e.g., silicon dioxide) contained in the porous carbon material precursor [1] or [2] is removed by an aqueous sodium hydroxide solution, heating the aqueous sodium hydroxide solution causes silicon dioxide to react as represented by the chemical formula (3) and to be removed as sodium silicate ($Na_2SiO_3$), whereby a porous carbon material can be obtained. In addition, in the case of treatment by reacting sodium hydroxide in a gaseous phase, heating sodium hydroxide in a solid state causes the sodium hydroxide to react as represented by the chemical formula (3) and to be removed as sodium silicate ($Na_2SiO_3$), whereby a porous carbon material can be obtained. It suffices that the porous carbon material is then washed and dried.

$$SiO_2 + 2NaOH \rightarrow Na_2SiO_3 + H_2O \qquad (3)$$

The adsorbent according to the present invention can be used to selectively adsorb the above-mentioned various unrequired molecules present in the living body. Furthermore, the adsorbent according to the present invention can be not only used as an adsorbent for adsorbing indole, an adsorbent for adsorbing uric acid, an adsorbent for adsorbing adenosine, an adsorbent for adsorbing $\alpha$-amylase, an adsorbent for adsorbing 3-methylindole, an adsorbent for adsorbing tryptophan, an adsorbent for adsorbing indican, an adsorbent for adsorbing theophylline, an adsorbent for adsorbing inosine 5-monophosphate disodium salt, an adsorbent for adsorbing a fatty acid (specifically, for example, oleic acid, stearic acid, myristic acid, squalene, or choresterol), an adsorbent for adsorbing a coloring matter (specifically, Lithol Rubine BCA), or an adsorbent for adsorbing hydrophobic molecules but also used as an adsorbent for adsorption of a water-soluble basic and amphoteric substance such as ammonia, urea, dimethylamine, guanidine compounds such as methylguanidine, etc., sulfur-containing amino acids, phenol, p-cresol, oxalic acid, homocysteine, guadininosuccinic acid, myo-inositol, indoxyl sulfate, pseudouridine, cyclic adenosine monophosphoric acid, creatinine, $\beta$-aminoisobutyric acid, octopamine, $\alpha$-aminobutyric acid, parathyroid hormone, $\beta$2-microglobulin, ribonuclease, nitriuretic hormone, aspartic acid, and arginine. Besides, the adsorbent used according to the present invention can also be used as an adsorbent for adsorption of purine or purine derivatives, adenine and guanine, which are purine bases, guanosine and inosine, which are purine nucleosides, and adenylic acid, guanylic acid and inosinic acid, which are purine nucleotides. Further, the adsorbent used according to the present invention can also be used as an adsorbent for adsorption of oligonucleotides and polynucleotides, which are low-molecular-weight or high-molecular-weight nucleic acids, and can be used as an adsorbent for adsorption of polyamines, 3-deoxyglucosone, various peptide hormones, granulocyte inhibitory proteins (GIP), degranulocyte inhibitory proteins (DIP), and chemical migration inhibitory proteins. Furthermore, the adsorbent used according to the present invention can also be used as an adsorbent for adsorption of carbamoylated hemoglobin, saccharification end products, granulocyte or monocyte inhibitor, oxidation promoter, etc. Or, the adsorbent used according to the present invention can also be used as an adsorbent for adsorption of 1,1-diphenyl-2-picrylhydrazyl (DPPH; number average molecular weight: 394), tyrosine (number average molecular weight: 394), and microcystins.

In the porous carbon material in the adsorbent, the cleansing agent, the renal disease drug or the functional food, the plant-derived material contains silicon in a content of not less than 5 wt.%. However, at the time of converting the plant-derived material to the porous carbon material precursor [1] or the porous carbon material by carbonizing the plant-derived material at 800 to 1400°C, the carbonization at a temperature in such a range ensures that silicon contained in the plant-derived material is not changed into silicon carbide (SiC) but turned into silicon components (silicon oxides) such as silicon dioxide ($SiO_X$), silicon oxide and silicon oxide salts. Therefore, the treatment with an acid or alkali (base) in the subsequent step removes the silicon components (silicon oxides) such as silicon dioxide, silicon oxide and silicon oxide salts, resulting in that a high value of specific surface area determined by the nitrogen BET method can be obtained. In the porous carbon material in the adsorbent, the cleansing agent, the renal disease drug or the functional food according to the present invention, the value of specific surface area determined by the nitrogen BET method is not less than 10 $m^2/g$, and the content of silicon is not more than 1 wt.%, and pore volumes determined by the BJH method and the MP method are not less than 0.1 $cm^3/g$, so that excellent functionality and characteristics can be obtained. This ensures that the porous carbon material in the present invention is optimum, for example, as a porous carbon material for removal of dirt components, as a porous carbon material for an adsorbent for oral administration, or as a porous carbon material aimed at adsorption of a protein or virus, and the above-mentioned substances can be effectively adsorbed by the adsorbent according to the present invention. In addition, renal disease drugs and functional foods having excellent characteristics can be provided.

Brief Description of Drawings

[FIG. 1]

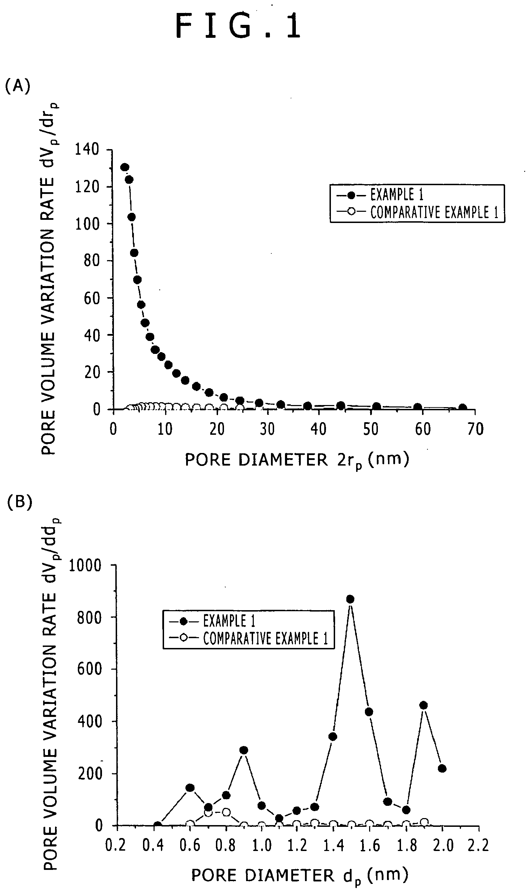

FIG. 1 shows, in (A) and (B), graphs representing pore size distribution of mesopores and pore size distribution of micropores in porous carbon materials in Example 1 and Comparative Example 1.
[FIG. 2]

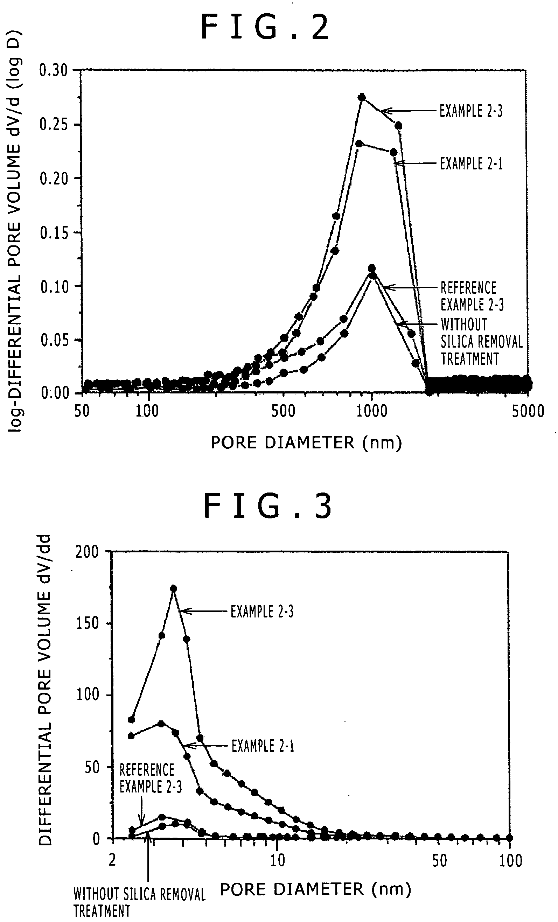

FIG. 2 is a graph showing the measurement results of pore size distribution determined by the mercury intrusion method, for porous carbon materials in Example 2-1, Example 2-3, Reference Example 2-3 and so on.
[FIG. 3]
FIG. 3 is a graph showing the measurement results of pore size distribution determined by the BJH method, for porous carbon materials in Example 2-1, Example 2-3, Reference Example 2-3 and so on.
[FIG. 4]

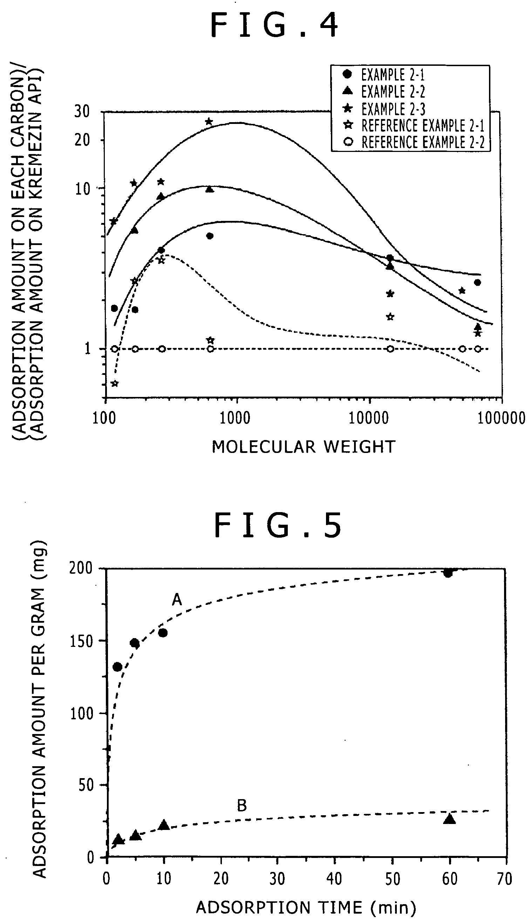

FIG. 4 is a graph showing normalized values of indole adsorption amount, uric acid adsorption amount, adenosine adsorption amount, and $\alpha$-amylase adsorption amount, where the adsorption amount per gram (1 g) of Kremezin API is taken as "1.0".
[FIG. 5]
FIG. 5 is a graph showing the results of examination of the relationship between adsorption amount and adsorption time in an aqueous solution of indole (aqueous solution A), in Example 2-3 and Reference Example 2-2.
[FIG. 6]

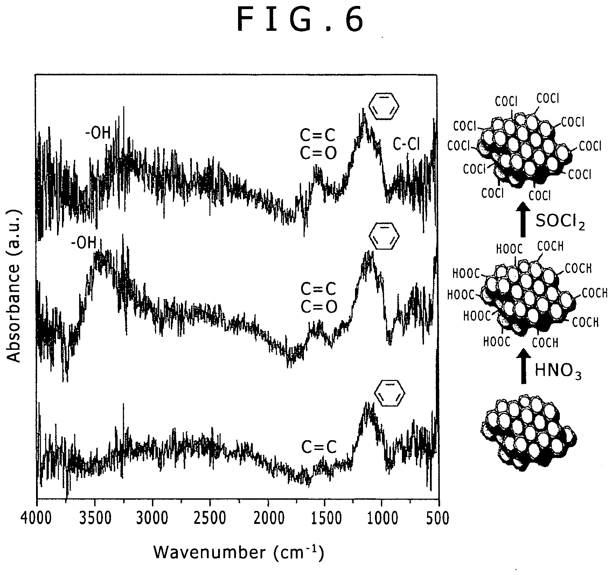

FIG. 6 is a graph showing the measurement results of infrared absorption spectrum of a porous carbon material in Example 3.
[FIG. 7]

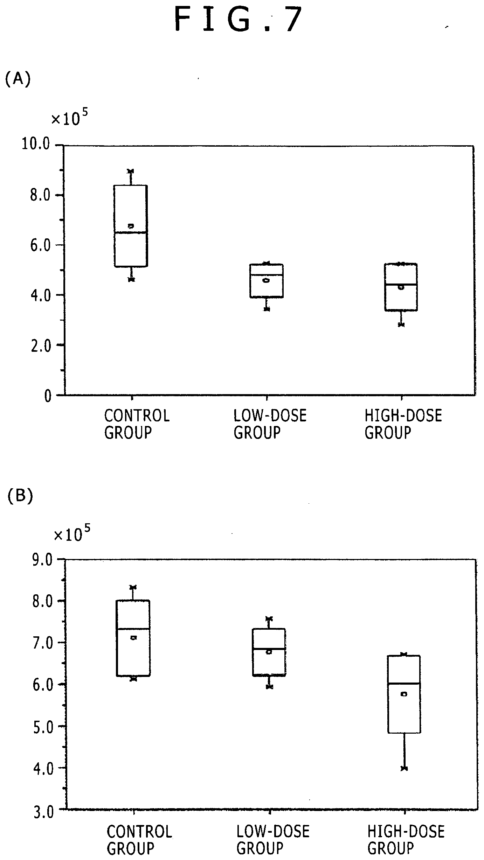

FIG. 7 shows, in (A) and (B), graphs showing the measurement results of contents of uric acid and creatinine in blood plasma of rats in Example 4.
[FIG. 8]

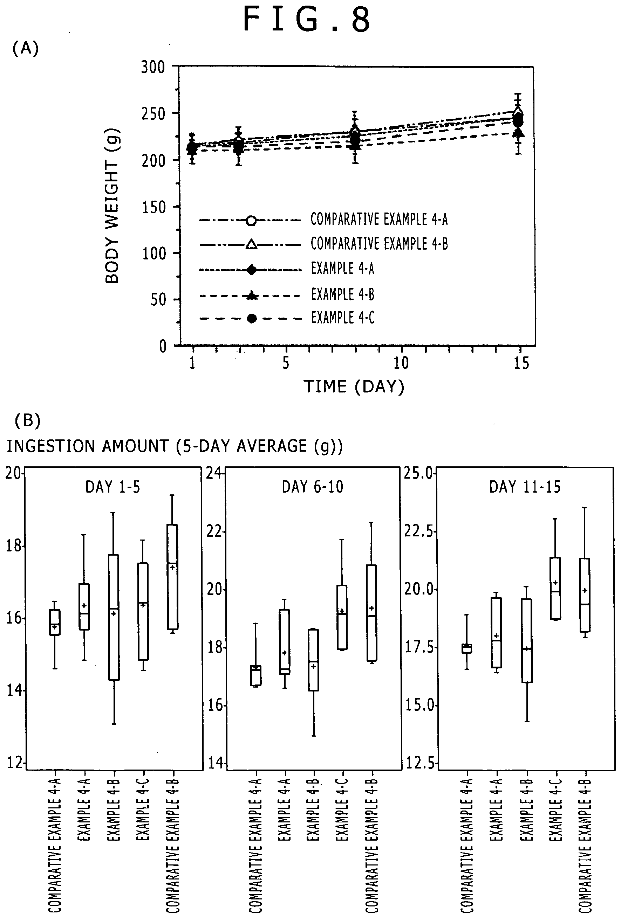

FIG. 8 shows, in (A) and (B), a graph showing time variation of body weight of rats in Example 4 and a graph showing average ingestion amount in Example 4.
[FIG. 9]

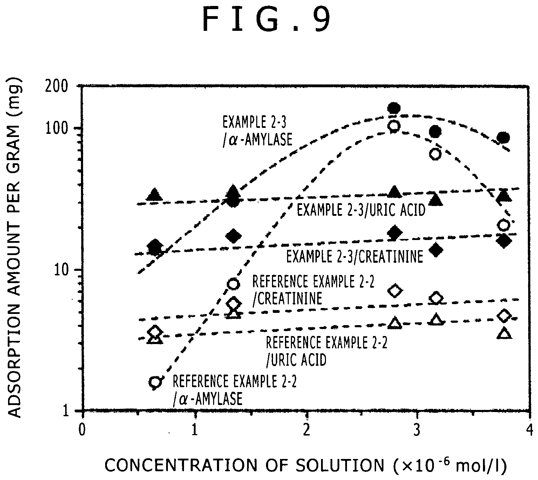

FIG. 9 is a graph showing the results of determination of adsorption amounts of nuric acid, creatinine and $\alpha$-amylase in Example 5.

Mode for Carrying Out the Invention

Now, the present invention will be described below, based on Examples and referring to the drawings. The present invention, however, is not limited to or by the Examples, and various numerical values and materials in the Examples are mere examples.

Example 1

First, in Example 1, description will be made of a porous carbon material used according to the present invention described in Example 2 later, the adsorbents for oral administration, and the cleansing agents, and a method for producing the porous carbon material.
In Example 1, the plant-derived material as a raw material for the porous carbon material is husks of rice. The porous carbon material in Example 1 is obtained by converting the rice husks as raw material into a carbonaceous material (porous carbon material precursor) by carbonization, and then treating the carbonaceous material with an acid.
In production of the porous carbon material, first, pulverized rice husks (rice husks of Isehikari rice produced in Kagoshima prefecture, Japan) was subjected to a heating treatment (pre-carbonizing treatment) in an inert gas.

Specifically, the rice husks was carbonized by heating in a nitrogen gas stream at 500°C for 5 hours, to obtain a carbonized matter. Incidentally, such a treatment makes it possible to reduce the amount of or eliminate tar components which would be produced in the subsequent carbonization. Thereafter, 10 g of the carbonized matter was placed in an alumina crucible, and, in a nitrogen gas stream (10 L/minute), temperature was raised to 1000°C at a temperature rise rate of 5°C/minute. Then, carbonization at 1000°C was conducted for 5 hours, to convert the carbonized matter into a carbonaceous material (porous carbon material precursor), followed by cooling to room temperature. Incidentally, the flow of nitrogen gas was continued during the carbonization and cooling. Next, the porous carbon material precursor was subjected to an acid treatment by immersing it in an aqueous 46 vol.% solution of hydrofluoric acid overnight, followed by washing by use of water and ethyl alcohol until a pH of 7 was reached. Finally, the washed product was dried, whereby a porous carbon material suitable for use in Examples 2 to 6 described later could be obtained.

Incidentally, a method may be adopted in which the pulverized rice husks is subjected to a heating treatment in an inert gas (pre-carbonizing treatment), and the heat-treated material is subjected to an acid treatment by immersing it in an aqueous 46 vol.% solution of hydrofluoric acid overnight, followed by carbonization to obtain a porous carbon material.

Incidentally, for comparison, the same raw material as that used in Example 1 was treated in the same manner as in Example 1, except for omission of the acid treatment, to obtain a porous carbon material (porous carbon material of Comparative Example 1).

The porous carbon materials of Example 1 and Comparative Example 1 were measured for specific surface area and pore volume, to obtain the results as shown in Table 1 below. In addition, the porous carbon materials of Example 1 and Comparative Example 1 were measured for pore diameter distribution of mesopores and micropores, to obtain the results as shown in (A) and (B) of FIG. 1.

As measuring apparatus for determination of specific surface area and pore volume, a nitrogen adsorption/desorption test was carried out by use of a measuring apparatus BELSORP-mini (made by BEL Japan, Inc.). As a measuring condition, a measurement equilibrium relative pressure ($p/p_0$) was set at a value in the range of 0.01 to 0.95. Then, specific surface area and pore volume were calculated, based on BELSORP analysis software. In addition, pore diameter distributions of mesopores and micropores were calculated by conducting the nitrogen adsorption/desorption test using the above-mentioned measuring apparatus and treating the test results on the basis of the BJH method and the MP method by use of the BELSORP analysis software.

As shown in Table 1, the specific surface area and pore volume of the porous carbon material of Example 1 having undergone the acid treatment were extremely high as compared with the specific surface area and pore volume of the porous carbon material of Comparative Example 1 obtained without the acid treatment. Specifically, the acid-treated porous carbon material of Example 1 had a specific surface area value of not less than 400 $m^2$/g and a pore volume value of not less than 0.1 $cm^3$/g. In addition, as shown in (A) of FIG. 1, the porous carbon material of Example 1 contained mesopores with a pore diameter of not more than 20 nm in a larger amount, particularly, mesopores with a pore diameter of not more than 10 nm in a larger amount, as compared with the porous carbon material of Comparative Example 1. Furthermore, as shown in (B) of FIG. 1, the porous carbon material of Example 1 contained micropores with a pore diameter of about 1.9 nm, micropores with a pore diameter of about 1.5 nm, and micropores with a pore diameter of about 0.8 nm to 1 nm in larger amounts, as compared with the porous carbon material of Comparative Example 1.

Besides, the porous carbon materials of Example 1 and Comparative Example 1 were served to elemental analysis, to obtain the results as shown in Table 2 below. Incidentally, an energy dispersive type X-ray analyzer (JED-2200F, made by JEOL Ltd.) was used as a measuring apparatus for the elemental analysis, and, after determination of elements by energy dispersive spectrometry (EDS), the contents of the elements were calculated in weight ratio (wt.%). The measuring conditions were a scanning voltage of 15 kV and an irradiation current of 13 μA.

As shown in Table 2, the porous carbon material of Example 1 having undergone the acid treatment showed reduced contents of silicon (Si), oxygen (O), potassium (K), calcium (Ca) and sodium (Na), as compared with the porous carbon material of Comparative Example 1 obtained without the acid treatment. Among the contents of the elements, the contents of silicon (Si) and oxygen (O) were extremely lower in Example 1 than in Comparative Example 1; specifically, the Si and O contents were not more than 1 wt.%. Besides, the contents of phosphorus (P) and sulfur (S) were higher in Example 1 than in Comparative Example 1. As a result, it was verified that the porous carbon material produced through the acid treatment after carbonization of rice husks as raw material at 800 to 1400°C has a silicon (Si) content of not more than 1 wt.%, a magnesium (Mg) content of 0.01 to 3 wt.%, a potassium (K) content of 0.01 to 3 wt.%, and a calcium (Ca) content of 0.05 to 3 wt.%. It was also confirmed that this porous carbon material has a phosphorus (P) content of 0.01 to 3 wt.% and a sulfur (S) content of 0.01 to 3 wt.%. Incidentally, though the kinds of elements are not shown as other elements, the amount of carbon (C) was the greatest of the amounts of the other elements; specifically, the amount of carbon (C) accounted for at least 90% of the total amount of the other elements. Here, silicon was contained in the rice husks as amorphous silica component, and the content of silicon in the rice husks used as raw material was 9.4 wt.%.

Besides, the porous carbon material of Example 1 was extremely lowered in silicon (Si) and oxygen (O) contents, as compared with the porous carbon material of Comparative Example 1. From this fact and also from the analytical results of Comparative Example 1, it was suggested that silicon dioxide had been contained in the carbonaceous material (porous carbon material precursor) in a large amount. It was thus suggested that the treatment of the porous carbon material precursor with an acid removes the silicon components such as silicon dioxide, and this removal contributes to an increase in the value of specific surface area. Further, it was confirmed that the treatment with an acid increases the amounts of mesopores and micropores. In addition, the same results as above were obtained also for porous carbon materials obtained through a treatment with an alkali (base) such as an aqueous solution of sodium hydroxide.

[Table 1]

| | Specific surface area ($m^2/g$) | Pore volume ($cm^3/g$) |
|---|---|---|
| Example 1 | 589 | 0.60 |
| Comp.Ex.1 | 6.26 | 0.018 |

[Table 2]

| | O | Na | Mg | Si | P | S | K | Ca | Others |
|---|---|---|---|---|---|---|---|---|---|
| Example 1 | <0.01 | <0.01 | 0.09 | 0.91 | 0.23 | 0.07 | 0.18 | 0.15 | 98.37 |
| Comp.Ex.1 | 22.77 | 0.05 | 0.08 | 19.10 | 0.03 | 0.02 | 0.59 | 0.20 | 57.16 |

Example 2

In relation to the adsorbents used according to the present invention, the adsorbent for oral administration and the cleansing agent, the porous carbon materials obtained in Example 1 were applied in Example 2 as a porous carbon material for selective adsorption of various unrequired molecules present in the living body, a porous carbon material for removal of dirt components, or a porous carbon material for an oral adsorbent. Then, adsorption amounts of various substances per unit weight of porous carbon material were measured.

In measuring the adsorption amount, first, 14 kinds of substances differing in number average molecular weight (number average M.W.), that is, indole (number average M.W.: 117), uric acid (number average M.W.: 168), adenosine (number average M.W.: 267), $\alpha$-amylase (number average M.W.: about 50000), 3-methylindole (number average M.W.: 131), theophylline (number average M.W.: 180), L-tryptophan (number average M.W.: 204), indicant (number average M.W.: 295), inosine 5-monophosphate disodium salt (number average M.W.: 392), adenosine 5-triphosphate disodium salt (number average M.W.: 551), oleic acid (number average M.W.: 282), squalene (number average M.W.: 411), cholesterol (number average M.W.: 387), Lithol Rubine BCA (number average M.W.: 424), and microcystin LR (number average M.W.: 994) were respectively used together with a phosphate buffer of PH 7.3, to prepare aqueous solutions (aqueous solution A, aqueous solution B, aqueous solution C, aqueous solution D, aqueous solution E, aqueous solution F, aqueous solution G, aqueous solution H, aqueous solution I, aqueous solution J, aqueous solution K, aqueous solution L, aqueous solution M, aqueous solution N, and aqueous solution O) having the respective concentrations set forth in Table 3 below. Incidentally, the concentration of each aqueous solution before adsorption was set in an arbitrary manner. Then, 0.010 g of the porous carbon material was added to 40.0 mL of each of the aqueous solutions prepared above, followed by shaking at $37\pm2°C$ for 1 hour. After the shaking, the porous carbon material was removed from each aqueous solution by use of a polytetrafluoroethylene-made membrane filter having a pore size of 500 $\mu$m. Then, light absorbance of each filtrate was measured by UV-visible absorbance measurement, to determine the molar concentration of the aqueous solution. Incidentally, by comparison of this with the initial molar concentration of the aqueous solution before adsorption, the adsorption amount of each substance (the amount of each substance adsorbed) was calculated. The adsorption amount per gram (1 g) of porous carbon material was calculated based on the following formula.

```
(Adsorption amount per gram of porous carbon material) =
    (Molecular weight of solute) × [(Molar concentration
    of aqueous solution before adsorption) - (Molar
    concentration of aqueous solution after adsorption)]/
    (Amount of porous carbon material per 1000 mL)
```

In Example 2, adsorbents as shown in Table 4 below were produced, based on the porous carbon material obtained in Example 1. Incidentally, Example 2-1 in Table 4 pertains to a porous carbon material produced by the same method as in Example 1 (except that the carbonizing temperature was 800°C and the carbonizing time was 1 hour); on the other hand, Example 2-2 and Example 2-3 pertain to porous carbon materials obtained by subjecting the porous carbon material of Example 1 to activating treatments shown in Table 4. Incidentally, in Example 2-2 and Example 2-3, steam (water vapor) was used as an activating agent, and the porous carbon material was heated in the water vapor atmosphere at 900°C for 2 hours and for 3 hours, respectively, so that a microstructure was developed by the volatile components and carbon molecules in the porous carbon material. In Table 4, the measurement results of specific surface area and the measurement results of pore volume are also shown. From Table 4, it is seen, by comparison between Example 2-2 and Example 2-3, that a longer activating time leads to greater increases in the value of specific surface area and the value of pore volume. The porous carbon materials of Example 2-1, Example 2-3, Reference Example 2-3 and Comparative Example 1 were served to measurement of pore size distribution by the mercury intrusion method and measurement of pore size distribution by the BJH method. In the pore size distribution determined by the mercury intrusion method, large peaks were present at about 1 $\mu$m (see FIG. 2); on the other hand, in the pore size distribution determined by the BJH method, large peaks were present at 4 nm (see FIG. 3). Incidentally, the data of Comparative Example 1 is indicated as "without silica removal treatment" in FIGS. 2 and 3.

Besides, for reference, in Reference Examples 2-1 and 2-2, measurement of adsorption amount per gram (1 g) of adsorbent was conducted using activated carbons set forth in Table 5 below.

[Table 3]

| | Solute | M.W. of solute | Molar concentration (mol/L) |
|---|---|---|---|
| Aq.Sol. A | Indole | 117 | $4.234 \times 10^{-4}$ |
| Aq.Sol. B | Uric acid | 168 | $4.616 \times 10^{-4}$ |
| Aq.Sol. C | Adenosine | 267 | $1.669 \times 10^{-4}$ |
| Aq.Sol. D | $\alpha$-Amylase | 50000 | $6.693 \times 10^{-6}$ |
| Aq.Sol. E | 3-Methylindole | 131 | $4.574 \times 10^{-4}$ |
| Aq.Sol. F | Theophylline | 180 | $2.959 \times 10^{-4}$ |
| Aq.Sol. G | L-Tryptophan | 204 | $7.609 \times 10^{-4}$ |
| Aq.Sol. H | Indican | 295 | $6.103 \times 10^{-4}$ |
| Aq.Sol. I | Inosine 5-monophosphate disodium salt | 392 | $4.136 \times 10^{-4}$ |
| Aq.Sol. J | Adenosine 5-triphosphate disodium salt | 551 | $2.141 \times 10^{-4}$ |
| Aq.Sol. K | Oleic acid | 282 | $5.07 \times 10^{-2}$ |
| Aq.Sol. L | Squalene | 411 | $5.03 \times 10^{-3}$ |
| Aq.Sol. M | Cholesterol | 387 | $5.01 \times 10^{-3}$ |
| Aq.Sol. N | Lithol Rubine BCA | 424 | $2.07 \times 10^{-5}$ |
| Aq.Sol. O | Microcystin LR | 994 | $1.00 \times 10^{-4}$ |
| Aq.Sol.: Aqueous solution | | | |

[Table 4]

| Example | Treatment after acid treatment | Specific surface area ($m^2$/g) | Pore volume ($cm^3$/g) |
|---|---|---|---|
| 2-1 | None | 589 | 0.60 |
| 2-2 | Steam activation 900°C $\times$ 2 hours | 930 | 0.80 |
| 2-3 | Steam activation 900°C $\times$ 3 hours | 1309 | 1.16 |

[Table 5]

| Reference Example | Product Name | Manufacturer | Raw material | Specific surface area (m²/g) | Pore volume (cm³/g) |
|---|---|---|---|---|---|
| 2-1 | Activated carbon (Kuraraycoal GW) | Kuraray Chemical Co. Ltd. | Coconut shell | 885 | 0.40 |
| 2-2 | Kremezin API | Kureha Corp. | Petroleum pitch | 1079 | 0.60 |
| 2-3 | Activated carbon | Wako Pure Chemical Industries, Ltd. | --- | 1231 | 0.57 |
| 2-4 | Medicinal carbon | Horie Shoyaku KK | --- | 924 | 0.70 |
| 2-5 | Medicinal carbon | Oriental Yakuhin Kogyo KK | --- | 928 | 0.76 |

Adsorption amount (g) of indole, adsorption amount (g) of uric acid, adsorption amount (g) of adenosine, adsorption amount (g) of $\alpha$-amylase, adsorption amount (g) of 3-methylindole, adsorption amount (g) of theophylline, adsorption amount (g) of L-tryptophan, adsorption amount (g) of indicant, adsorption amount (g) of inosine 5-monophosphate disodium salt, adsorption amount (g) of adenosine 5-triphosphate disodium salt, microcystin LR (g), adsorption amount (g) of oleic acid, adsorption amount (g) of squalene, adsorption amount (g) of cholesterol, and adsorption amount (g) of Lithol Rubine BCA, per gram (1 g) of the porous carbon material or each of the activated carbons, are shown in Tables 6, 7, 8, 9, 10, 11, 12, 13, 14, 15, 16, 17, 18, 19, and 20 below.

[Table 6]

Adsorption amount (mg) of indole per milligram of porous carbon material or the like
Number average M.W. of indole: 117

| | Adsorption amount of indole (mg) | Specific surface area (m²/g) | Pore volume (cm³/g) |
|---|---|---|---|
| Example 2-1 | 58.98 | 589 | 0.60 |
| Example 2-2 | 167.25 | 930 | 0.80 |
| Example 2-3 | 205.81 | 1309 | 1.16 |
| Ref.Ex. 2-1 | 24.10 | 885 | 0.40 |
| Ref.Ex. 2-2 | 32.88 | 1079 | 0.60 |

[Table 7]

Adsorption amount (mg) of uric acid per milligram of porous carbon material or the like
Number average M.W. of uric acid: 168

| | Adsorption amount of uric acid (mg) | Specific surface area (m²/g) | Pore volume (cm³/g) |
|---|---|---|---|
| Example 2-1 | 24.34 | 589 | 0.60 |
| Example 2-2 | 76.14 | 930 | 0.80 |
| Example 2-3 | 150.10 | 1309 | 1.16 |
| Ref.Ex. 2-1 | 36.94 | 885 | 0.40 |
| Ref.Ex. 2-2 | 13.86 | 1079 | 0.60 |

[Table 8]

| | Adsorption amount of adenosine (mg) | Specific surface area (m²/g) | Pore volume (cm³/g) |
|---|---|---|---|
| Adsorption amount (mg) of adenosine per milligram of porous carbon material or the like<br>Number average M.W. of adenosine: 267 | | | |
| Example 2-1 | 55.42 | 589 | 0.60 |
| Example 2-2 | 119.59 | 930 | 0.80 |
| Example 2-3 | 149.12 | 1309 | 1.16 |
| Ref.Ex. 2-1 | 48.07 | 885 | 0.40 |
| Ref.Ex. 2-2 | 13.45 | 1079 | 0.60 |

[Table 9]

| | Adsorption amount of $\alpha$-amylase (mg) | Specific surface area (m²/g) | Pore volume (cm³/g) |
|---|---|---|---|
| Adsorption amount (mg) of $\alpha$-amylase per milligram of porous carbon material or the like<br>Number average M.W. of $\alpha$-amylase: 50000 | | | |
| Example 2-3 | 86.92 | 1309 | 1.16 |
| Ref.Ex. 2-2 | 37.84 | 1079 | 0.60 |

[Table 10]

| | Adsorption amount of 3-methylindole (mg) |
|---|---|
| Adsorption amount (mg) of 3-methylindole per milligram of porous carbon material or the like<br>M.W. of 3-methylindole: 131 | |
| Example 2-2 | 187.45 |
| Example 2-3 | 227.08 |
| Ref.Ex. 2-2 | 117.08 |

[Table 11]

| | Adsorption amount of theophylline (mg) |
|---|---|
| Adsorption amount (mg) of theophylline per milligram of porous carbon material or the like<br>M.W. of theophylline: 180 | |
| Example 2-1 | 52.81 |
| Example 2-2 | 140.38 |
| Example 2-3 | 199.35 |
| Ref.Ex. 2-1 | 48.58 |
| Ref.Ex. 2-2 | 38.39 |

[Table 12]

| | Adsorption amount of L-tryptophan (mg) |
|---|---|
| Adsorption amount (mg) of L-tryptophan per milligram of porous carbon material or the like<br>M.W. of L-tryptophan: 204 | |
| Example 2-3 | 262.46 |

(continued)

| Adsorption amount (mg) of L-tryptophan per milligram of porous carbon material or the like M.W. of L-tryptophan: 204 | |
|---|---|
| | Adsorption amount of L-tryptophan (mg) |
| Ref.Ex. 2-1 | 68.28 |
| Ref.Ex. 2-2 | 111.63 |

[Table 13]

| Adsorption amount (mg) of indicant per milligram of porous carbon material or the like M.W. of indicant: 295 | |
|---|---|
| | Adsorption amount of indicant (mg) |
| Example 2-2 | 164.03 |
| Example 2-3 | 291.9 |
| Ref.Ex. 2-1 | 46.93 |
| Ref.Ex. 2-2 | 148.50 |

[Table 14]

| Adsorption amount (mg) of inosine 5-monophosphate disodium salt per milligram of porous carbon material or the like M.W. of inosine 5-monophosphate disodium salt: 392 | |
|---|---|
| | Adsorption amount of inosine 5-monophosphate disodium salt (mg) |
| Example 2-2 | 118.95 |
| Example 2-3 | 354.39 |
| Ref.Ex. 2-1 | 56.46 |
| Ref.Ex. 2-2 | 68.89 |

[Table 15]

| Adsorption amount (mg) of adenosine 5-triphosphate disodium salt per milligram of porous carbon material or the like M.W. of adenosine 5-triphosphate disodium salt: 551 | |
|---|---|
| | Adsorption amount of adenosine 5-triphosphate disodium salt (mg) |
| Example 2-3 | 213.30 |
| Ref.Ex. 2-1 | 8.54 |
| Ref.Ex. 2-2 | 47.79 |

[Table 16]

| Adsorption amount (mg) of microcystin LR per gram of porous carbon material or the like M.W. of microcystin LR: 994 | |
|---|---|
| | Adsorption amount of micro-cystine LR (mg) |
| Example 2-3 | 271 |

(continued)

| Adsorption amount (mg) of microcystin LR per gram of porous carbon material or the like M.W. of microcystin LR: 994 | |
|---|---|
| | Adsorption amount of micro-cystine LR (mg) |
| Ref.Ex. 2-3 | 21 |

[Table 17]

| Adsorption amount (g) of oleic acid per gram of porous carbon material or the like M.W. of oleic acid: 282 | |
|---|---|
| | Adsorption amount of oleic acid (g) |
| Example 2-3 | 4.14 |
| Ref.Ex. 2-3 | 2.91 |

[Table 18]

| ] Adsorption amount (g) of squalene per gram of porous carbon material or the like M.W. of squalene: 411 | |
|---|---|
| | Adsorption amount of squalene (g) |
| Example 2-3 | 0.45 |
| Ref.Ex. 2-4 | 0.32 |
| Ref.Ex. 2-5 | 0.30 |

[Table 19]

| Adsorption amount (g) of cholesterol per gram of porous carbon material or the like M.W. of cholesterol: 387 | |
|---|---|
| | Adsorption amount of cholesterol (g) |
| Example 2-3 | 0.71 |
| Ref.Ex. 2-3 | 0.49 |
| Ref.Ex. 2-4 | 0.44 |
| Ref.Ex. 2-5 | 0.39 |

[Table 20]

| Adsorption amount (g) of Lithol Rubine BCA per gram of porous carbon material or the like M.W. of Lithol Rubine BCA: 424 | |
|---|---|
| | Adsorption amount of Lithol Rubine BCA (g) |
| Example 2-3 | 0.27 |
| Ref.Ex. 2-3 | 0.16 |
| Ref.Ex. 2-4 | 0.19 |
| Ref.Ex. 2-5 | 0.19 |

From Table 6 it is seen that in Examples 2-1, 2-2 and 2-3, the adsorption amount of indole per gram (1 g) of porous carbon material tends to increase with increases in the value of specific surface area of the porous carbon material and the value of pore volume in the material, with good correlation therebetween. In addition, every one of Examples 2-1, 2-2 and 2-3 showed a higher indole adsorption amount as compared with Reference Examples 2-1 and 2-2.

Besides, from Table 7 it is seen that in Examples 2-1, 2-2 and 2-3, the adsorption amount of uric acid per gram (1 g) of porous carbon material tends to increase with increases in the value of specific surface area of the porous carbon material and the value of pore volume in the material, with good correlation therebetween. In addition, Examples 2-2 and 2-3 showed a higher uric acid adsorption amount as compared with Reference Examples 2-1 and 2-2.

Further, from Table 8 it is seen that in Examples 2-1, 2-2 and 2-3, the adsorption amount of adenosine tends to increase with increases in the value of specific surface area of the porous carbon material and the value of pore volume in the material, with good correlation therebetween. In addition, every one of Examples 2-1, 2-2 and 2-3 showed a higher adenosine adsorption amount as compared with Reference Examples 2-1 and 2-2.

Besides, it is seen from Table 9 that the adsorption amount of $\alpha$-amylase was higher in Example 2-3 than in Reference Example 2-2.

Further, from Table 10 it is seen that in Examples 2-2 and 2-3, the adsorption amount of 3-methylindole per gram (1 g) of porous carbon material tends to increase with increases in the value of specific surface area of the porous carbon material and the value of pore volume in the material. In addition, Examples 2-2 and 2-3 showed a higher 3-methylindole adsorption amount as compared with Reference Example 2-2.

Besides, from Table 11 it is seen that in Examples 2-1, 2-2 and 2-3, the adsorption amount of theophylline per gram (1 g) of porous carbon material tends to increase with increases in the value of specific surface area of the porous carbon material and the value of pore volume in the material, with good correlation therebetween. In addition, every one of Examples 2-1, 2-2 and 2-3 showed a greater theophylline adsorption amount as compared with Reference Examples 2-1 and 2-2.

Further, it is seen from Table 12 that the adsorption amount of L-tryptophan was higher in Example 2-3 than in Reference Examples 2-1 and 2-2.

In addition, it is seen from Table 13 that the adsorption amount of indicant was greater in Examples 2-2 and 2-3 than in Reference Examples 2-1 and 2-2.

Further, from Table 14 it is seen that in Examples 2-2 and 2-3, the adsorption amount of inosine 5-monophosphate disodium salt per gram (1 g) of porous carbon material tends to increase with increases in the value of specific surface area of the porous carbon material and the value of pore volume in the material. In addition, the adsorption amount of inosine 5-monophosphate disodium salt was greater in Examples 2-1 and 2-3 than in Reference Examples 2-1 and 2-2.

Besides, it is seen from Table 15 that the adsorption amount of adenosine 5-triphosphate disodium salt was higher in Example 2-3 than in Reference Examples 2-1 and 2-2.

From Table 16 it is seen that Example 2-3 was greater than Reference Example 2-3 in adsorption amount of microcystin.

Further, it is seen from Table 17 that the adsorption amount of oleic acid was greater in Example 2-3 than in Reference Example 2-3.

In addition, it is seen from Table 18 that the adsorption amount of squalene was greater in Example 2-3 than in Reference Examples 2-4 and 2-5.

Further, it is seen from Table 19 that the adsorption amount of cholesterol was greater in Example 2-3 than in Reference Examples 2-3, 2-4 and 2-5.

In addition, from Table 20 it is seen that the adsorption amount of Lithol Rubine BCA was larger in Example 2-3 than in Reference Examples 2-3, 2-4 and 2-5.

Based on the results shown in Tables 6 to 9, the values of the adsorption amount of indole, the adsorption amount of uric acid, the adsorption amount of adenosine and the adsorption amount of $\alpha$-amylase were normalized, with the adsorption amount per gram (1 g) of Kremezin API in Reference Example 2-2 being taken as "1.0." The normalized values of adsorption amount are shown in FIG. 4 and Table 21. Incidentally, test results for Alizarine Cyanine Green (number average M.W.: 623) and lysozyme (number average M.W.: 14307) are added to FIG. 4, for reference. From FIG. 4, it is seen that the adsorbents in Examples 2 show effective adsorption of, particularly, organic matters having a number average M.W. of not more than $1 \times 10^2$ and less than $5 \times 10^2$. In addition, based on the results shown in Tables 10 to 15, the values of adsorption amount of 3-methylindole, the adsorption amount of theophylline, the adsorption amount of L-tryptophan, the adsorption amount of indicant, the adsorption amount of inosine 5-monophosphate disodium salt, and the adsorption amount of adenosine 5-triphosphate disodium salt were normalized, with the adsorption amount per gram (1 g) of Kremezin API in Reference Example 2-2 being taken as "1.0." The normalized values of adsorption amount are shown in Table 22.

[Table 21]

|  | Adsorption amount of indole | Adsorption amount of nuric acid | Adsorption amount of adenosine | Adsorption amount of $\alpha$-amylase |
|---|---|---|---|---|
| Number average M.W. | 117 | 168 | 267 | 50000 |
| Example 2-1 | 1.8 | 1.8 | 4.1 | |
| Example 2-2 | 5.1 | 5.5 | 8.9 | |
| Example 2-3 | 6.3 | 10.8 | 11.1 | 2.3 |

[Table 22]

|  | 3-Methyl-indole | Theophylline | L-Tryptophan | Indican | Inosine 5-monophosphate disodium salt | Adenosine 5-triphosphate disodium salt |
|---|---|---|---|---|---|---|
| Number average M.W. | 131 | 180 | 204 | 295 | 392 | 551 |
| Example 2-1 | | 1.4 | | | | |
| Example 2-2 | 1.6 | 3.7 | | 1.1 | 1.7 | |
| Example 2-3 | 1.9 | 5.2 | 2.4 | 2.0 | 5.1 | 4.5 |

In addition, the results of examination of relationship between adsorption amount and adsorption time in an aqueous solution of indole (aqueous solution A) in Example 2-3 and Example 2-2 are shown in FIG. 5, as [A] (Example 2-3) and [B] (Example 2-2). It is seen from the figure that Example 2-3 showed a greater adsorption amount of indole in a shorter time, as compared with Example 2-2.

As has been described above, it was found that adsorption characteristics of molecules on the porous carbon materials differ depending on the differences in parameters such as specific surface area and pore volume of the porous carbon material, differences in physical surface conditions and/or chemical surface conditions of the porous carbon material, and differences in chemical interaction between the porous carbon material and the adsorbate. It was also found that there is difference between the behavior of the porous carbon material as to adsorption thereon of low-molecular-weight molecules and the behavior of the porous carbon material as to adsorption thereon of high-molecular-weight molecules. Specifically, it was found that the porous carbon material used according to the present invention is higher than the activated carbons of Reference Examples in capability of adsorbing the low-molecular-weight molecules. Therefore, by determining the relationship between the molecular weight of molecules to be adsorbed and the parameters such as specific surface area and pore volume of the porous carbon material and the relationship between the molecular weight and the production method of the porous carbon material and the like, it is possible to achieve selective adsorption of molecules by the porous carbon material, which is expected to show a great effect in various medical uses where adsorption is required.

In lakes, ponds or marshes, abnormal propagation of blue-green algae (Microcystis, etc.) occurs in and around summery season, to form a thick layer appearing as if the water surface became powder-coated, which is called water bloom. The blue-green algae are known to produce toxins harmful to the human body. Among the many toxins, a toxin called microcystin LR is an object of special warning. When microcystin LR has entered the human body, the liver is damaged heavily; the toxicity of microcystin LR has also been reported as a result of experiments conducted using mice. The toxic water bloom producing microcystin LR is generated in lakes in Australia, Europe and America as well as many places in Asia. In a Chinese lake suffering serious damages, the water bloom after an outbreak remains non-disappearing all the year round. Since lake water is utilized as drinking water and agricultural water, the toxins produced by blue-green algae in lakes, ponds or marshes is causing problems in connection with securing drinking water for human beings, and the solution of the problem is being keenly desired. The adsorbent used according to the present invention has a high ability to adsorb microcystin, as above-described. Accordingly, by use of the adsorbent, the water bloom and microcystins contained as toxic components in lake water or river water and the like can be adsorbed easily, securely and economically.

Example 3

Example 3 is a modification of Example 2. In Example 3, the surface of the porous carbon material of Example 2-1 described in Example 2 was chemically modified with organic molecules.

Specifically, using a 500-mL eggplant-shaped flask, 3.0 g of the porous carbon material of Example 2-1 and 300 mL of concentrated nitric acid were mixed with each other, and the mixture was stirred at room temperature for 12 hours. Thereafter, the resulting carbon material was washed with pure water. For the porous carbon material thus obtained (referred to as the porous carbon material of Example 3-A), a peak indicative of carboxyl group (C=O: 1500 $cm^{-1}$) and a peak indicative of hydroxyl group (-OH: 3700 $cm^{-1}$) could be observed by IR spectrometry, whereby the presence of carboxyl groups could be confirmed. FIG. 6 shows the IR absorption spectra, in which the IR absorption spectrum shown at the bottom of FIG. 6 is the IR absorption spectrum for the porous carbon material of Example 2-1, and the IR absorption spectrum shown in the middle of FIG. 6 is the IR absorption spectrum for the porous carbon material of Example 3-A.

Further, 2.5 g of the porous carbon material of Example 3-A and 37.5 mL of thionyl chloride were mixed with each other, so as to achieve chlorination. For the porous carbon material thus obtained (referred to as the porous carbon material of Example 3-B), the peak indicative of hydroxyl group was reduced and a peak indicative of only C=O was mainly observed, as seen from the IR absorption spectrum. This is considered to be attributable to replacement of hydroxyl group by chlorine (see the IR absorption spectrum shown at the top of FIG. 6).

Incidentally, in all the processes, a peak indicative of the double bonds of benzene ring were observed.

Using the porous carbon materials of Example 3-A and Example 3-B obtained above as adsorbent, adsorption of each of indole, uric acid, adenosine, α-amylase, 3-methylindole, L-tryptophan, indicant, theophylline, inosine 5-monophosphate disodium salt, and adenosine 5-triphosphate disodium salt onto the adsorbents was conducted. It was found that the porous carbon materials of Example 3-A and Example 3-B adsorbed these substances (adsorbates) in larger amounts, as compared with the adsorbent of Reference Example 2-2.

Example 4

Example 4 relates to the renal disease drug use of a porous carbon material which is produced from a plant-derived material having a silicon content of not less than 5 wt.% and which has a value of specific surface area determined by the nitrogen BET method of not less than 10 $m^2/g$, a silicon content of not more than 1 wt.% and pore volumes determined by the BJH method and the MP method of not less than 0.1 $cm^3/g$. Specifically, the porous carbon material is composed of the porous carbon material described in Example 2-3 above.

The form in which this porous carbon material is to be administered may be any of powder, granule, tablet, sugar-coated tablet, capsule, suspension, emulsion, etc. In the case where the porous carbon material is to be ingested as a capsule drug, a capsule formed from ordinary gelatin or other enteric material may be used. Where a tablet form is adopted, an excipient such as lactose, starch, etc., a binder such as hydroxypropyl cellulose, acacia paste, starch paste, etc., a wax such as magnesium stearate, etc., a lubricant such as talc, etc., a disintegrator such as celluloses, etc. and so on may be used. Furthermore, the porous carbon material may be administered in the form of a composite medicine with alumina or silica component.

Using female rats (species: SPF/lineage: Crl:CD(SD), body weight: 150 to 220 g, supplied from Charles River Laboratories Japan, Inc.), ligation of the left hilum of kidney was conducted under anesthesia with isoflurane, and removal of the kidney was carried out. After 10 days, ligation of the right hilum of kidney was conducted, and removal of the kidney was carried out. As a result, rats as renal failure model with the hilums of kidney on both sides ligated were obtained, which were served to tests. Such rats were prepared in three 10-membered groups. Incidentally, the rats were supplied when they were 6 weeks old. Feeding was conducted by a method in which the feed is placed in a stainless steel-made feeder so that the rats can take the feed freely. As for water feeding, free drinking was permitted by use of an automatic water feeder. For rats not accustomed to the automatic water feeding, a glass-made water feeder equipped with a touch-drink type tip pipe was also used. In addition, the breeding conditions were as follows.

| Temperature: | 23°C |
| Humidity: | 55% RH |
| Ventilation frequency: | 16 times/hour |

(continued)

| Illumination time: | 12 hours/day (artificial illumination from 7:00 to 19:00) |
|---|---|

Administration of the renal disease drug was started when the rats were 9 weeks old. At the time of starting the administration, the body weights of the rats were in the range of 150 to 240 g. The method of administering the renal disease drug was as follows. Mixtures prepared by mixing various porous carbon materials with water in predetermined ratios were administered to the rats in the groups. Specifically, a test material-containing liquid or a control (reference) material-containing liquid prepared on the basis of each individual was sucked into a 10-mL syringe, while stirring the liquid. After the carbon material in the syringe was dispersed by a vortex mixer, the dispersed liquid was swiftly administered forcibly and orally into the stomach by use of a rat sound. Incidentally, distilled water was administered forcibly and orally into the rats in the control group. Details of the administration to the rats in each of the groups were as given in Table 23 below. Here, the number of times of administration was once a day, and the administration hour was 9:00 to 13:00. It is to be noted that the administration was started after the renal-failure models were made.

After the experiment was started, the living/dead states of the models were recorded, and, when a rat in a moribund state was found, the rat was euthanized. All the rats in each group were alive until 24 hours passed from the start of observation. However, after the lapse of 36 hours from the start of observation, the group dosed with the porous carbon material described in Example 2-3 was found to be higher in survival rate than the control group. In addition, from the number of deaths in each of observation times (see Table 24), the median (see Table 25) for the group dosed with the porous carbon material described in Example 2-3 was on the longer-time side, as compared with that for the control group. From these results, it was verified that the renal disease drug according to the present invention is effective in restraining a deterioration of the renal-disease condition. Incidentally, the bracketed numbers in Table 24 represent the medians.

[Table 23]

| Group | Administered material | Dose | |
|---|---|---|---|
| | | g/rat/day | mL/rat/day |
| Low-dose group | Example 2-3 | 0.5 | 7 |
| High-dose group | Example 2-3 | 1.0 | 7 |
| Control group | Distilled water | --- | 7 |

[Table 24]

| Observation time (hour) | 0 | 24 | 36 | 48 | 60 | 72 | 84 |
|---|---|---|---|---|---|---|---|
| Low-dose group | 0 | 0 | 1 | 4 | [3] | 2 | 0 |
| High-dose group | 0 | 0 | 0 | 5 | [4] | 1 | 0 |
| Control group | 0 | 0 | 1 | [6] | 3 | 0 | 0 |

[Table 25]

| Observation time (hour) | Median of number of deaths in each observation time (hour) |
|---|---|
| Low-dose group | 60 |
| High-dose group | 60 |
| Control group | 48 |

The blood plasma of each rat was sampled, the sample was served to high performance liquid chromatograph (HPLC) measurement, and comparison of uric acid and creatinine contents between the groups was conducted. Respective peak areas were analyzed. The measuring conditions in the HPLC were as follows.

[Measuring conditions]

| | |
|---|---|
| Sampling capacity: | 80 μL |
| Column: | polyhydroxy methacrylate |
| Effluent: | pH 7.4 phosphate buffer |
| Efflux rate: | 1.0 mL/minute |
| Detection: | UV rays 254 nm |
| Determination method: | peak area |
| Analysis time: | about 20 minutes |

Box plots of the measurement results are shown in (A) and (B) of FIG. 7. Incidentally, (A) and (B) in FIG. 7 show peak areas of uric acid and creatinine in the samples taken in the afternoon on the third day of administration. The group dosed with the renal disease drug of Example 4 was found to show lowered concentrations of uric acid and creatinine in blood, as compared with the control group. Especially, conspicuous lowering of concentration in blood was observed as to uric acid in the low-dose group and the high-dose group, and as to creatinine in the high-dose group. Accordingly, the efficacy of the renal disease drug of Example 4 was recognized.

By mixing the renal disease drug of Example 4 into the feed, a feeding test was conducted using rats. Specifically, mixed feeds were prepared based on the following method.

The renal disease drug of Example 4 as a test substance and a control (reference) substance were mixed with a powder feed by use of a dry type powder mixer. Here, in preparing mixed feeds respectively containing 1 wt.%, 5 wt.% and 10 wt.% of the test substance, the test substance was weighed in respective amounts of 10 g, 50 g and 100 g in relation to the total amount of each mixed feed of 1.0 kg. On the other hand, the control substance was weighed in an amount of 50 g, for preparing a mixed feed containing the control substance in an amount of 5 wt.% based on the total amount of mixed feed of 1.0 kg. Then, the test substance and the control substance were mixed with a small amount of the powder feed by placing in a sealable polyethylene bag. Next, each of the mixtures thus obtained was transferred into a drum of the dry type powder mixer, and an operation of adding a small amount of the powder feed at a time followed by mixing was repeated three or four times, as a pre-treatment. Then, the residual amount of the powder feed was placed into the drum, and the dry type powder mixer was operated for 30 minutes. Thereafter, the mixed powder feed was taken out of the drum. The mixed powder feeds prepared in this manner were placed in sealed containers on the basis of each preparation concentration, to be used as test feed.

Female rats (species: SPF/lineage: Crl:CD (SD), supplied from Charles River Laboratories Japan Inc., Hino Breeding Center) were prepared for use. Incidentally, the rats were female by sex, and 33 rats were used for the test. The rats were supplied when they were 8 weeks old. Starting from the day of supply of the rats, they were subjected to a quarantine inspection for 6 days. During the quarantine period, general conditions of the rats were observed everyday, and body weight measurement was conducted on the first day (the day of supply), the third day, and the seventh day (the day when the quarantine was over). In addition, the period from the day of supply to the day before the administration starting day was set as a training period, during which general conditions of the rats were observed once a day. The administration was started when the rats were 9 weeks old. At the time of starting the administration, the body weights of the rats were in the range of 150 to 240 g. In addition, the same route as the clinical administration route was selected, and the administration period was set to be 14 days. Feeding was conducted by a method in which the feed is placed in a stainless steel-made feeder so that the rats can take the feed freely. As for water feeding, free drinking was permitted by use of an automatic water feeder. For rats not accustomed to the automatic water feeding, a glass-made water feeder equipped with a touch-drink type tip pipe was also used. The breeding conditions were as follows. Besides, the constitutions of the test groups were as set forth below.

| | |
|---|---|
| Temperature: | 23°C |
| Humidity: | 55% RH |
| Ventilation frequency: | 16 times/hour |
| Illumination time: | 12 hours/day (artificial illumination from 7:00 to 19:00) |

| Group | Test feed | Concentration in mixed feed (wt.%) | Number of rats |
|---|---|---|---|
| Example 4-A | Example 4 | 1 | 6 |
| Example 4-B | Example 4 | 5 | 6 |
| Example 4-C | Example 4 | 10 | 6 |
| Comp.Ex. 4-A | Medium (powder feed) | -- | 6 |

(continued)

| Group | Test feed | Concentration in mixed feed (wt.%) | Number of rats |
|---|---|---|---|
| Comp.Ex. 4-B | Kremezin | 5 | 6 |

As a result of the test, excretion of a black stool considered to contain the test feed was observed in Example 4-A group, Example 4-B group, Example 4-C group, Comparative Example (Comp.Ex.) 4-A group, and Comparative Example 4-B group. It was confirmed that the substance mixed into the feed was excreted together with the ingested feed, starting from the day after the mixed-feed feeding starting day. Variations in body weight with time are shown in (A) of FIG. 8. The body weight on the 14th day of administration (Day 15) was a little smaller in Example 4-B group and Example 4-C group than in Comparative Example 4-A group. However, a tendency that the body weight would decrease with an increase in the concentration of the test substance mixed into the feed (concentration in mixed feed) was not recognized. Therefore, the decrease in the body weight is not considered to be a bad influence of the ingestion of the test substance.

Average ingestion amounts are shown in (B) of FIG. 8. The condition of ingestion of the mixed feed was good in both Examples and Comparative Examples. The ingestion amounts in Example 4-A group and Example 4-B group were approximately equal to that in Comparative Example 4-A group. The ingestion amounts in Example 4-C group and Comparative Example 4-B group were a little greater than that in Comparative Example 4-A group. In these groups in which the ingestion amount was large, the amount of stool was also increased, as compared with Comparative Example 4-A group. Therefore, it was inferred that the ingestion of the feed admixed with the renal disease drug of Example 4 had not produced an influence on the stool. In addition, upon autopsy conducted when the observation period was over, in each concentration group of Example 4-A group, Example 4-B group, Example 4-C group and Comparative Example 4-B group, a black-colored matter considered to be the test substance was observed in the ileum and the large intestine, which are the same parts as those in which the digest was found in Comparative Example 4-A group. Thus, it was confirmed that the test substance had not stagnated in any specific part of the rat body. From these results, it is concluded that when rats are fed with the renal disease drug of Example 4 in concentrations of 1 to 10 wt.% under the test conditions of Example 4, normal ingestion is observed and breeding can be performed without any bad influence on the living body.

Example 5

In Example 5, five kinds of mixed solutions containing uric acid, creatinine and $\alpha$-amylase mixed in the concentrations shown in Table 26 below were prepared, and adsorption amounts of the respective molecules of the substances were determined. Incidentally, the adsorbent of Example 2-3 and the adsorbent of Reference Example 2-2 were used as the adsorbent here. The results are shown in FIG. 9. From these results, it was confirmed that the adsorbent of Example 2-3 is greater in adsorption amount and higher in selective adsorption characteristic for uremic toxin (uric acid, creatinine), as compared with the adsorbent of Reference Example 2-2.

[Table 26]

| | | | Unit: mol/L |
|---|---|---|---|
| Mixed solution | Uric acid | Creatinine | $\alpha$-Amylase |
| 1 | $7.0\times10^{-5}$ | $9.3\times10^{-5}$ | $3.8\times10^{-6}$ |
| 2 | do. | do. | $3.2\times10^{-6}$ |
| 3 | do. | do. | $2.7\times10^{-6}$ |
| 4 | do. | do. | $1.4\times10^{-6}$ |
| 5 | do. | do. | $6.5\times10^{-7}$ |

Example 6

Example 6 relates to the functional food use according to the present invention. In Example 6, based on the porous carbon materials (Example 2-1, Example 2-2 and Example 2-3) described in Example 2 above, functional foods containing these porous carbon materials were produced. Specifically, the functional foods were produced based on a method in which, for example, the porous carbon material, a microcrystalline cellulose preparation obtained by coating microcrystalline cellulose with carboxymethyl cellulose sodium, a sweetening agent, and a seasoning agent were mixed, and the resulting mixture is dispersed in water, followed by kneading and molding (shaping) or the like.

While the case of using rice husks (chaff) as raw material for the porous carbon material has been described in Examples above, the raw material to be used here may be straw, common reed, *kukiwakame* seaweed or, further, other plants. Examples of the other plants include vascular plant (Tracheophyta) vegetating on land, Pteridophyta, Bryophyta, algae and seaweed, which may be used either singly or in mixture of some of them.

Besides, in relation to the porous carbon material used according to the present invention, description has been made of the appropriate ranges for the specific surface area based on the nitrogen BET method and for the contents of various elements. Specifically, the above-mentioned appropriate ranges are only ranges which are particularly preferable for obtaining the effect of the present invention; accordingly, the value of the specific surface area and the like may be slightly outside the above-mentioned ranges insofar as the effect of the invention can be obtained.

In addition, the porous carbon material used according to the present invention can be used as a cell culture material (cell culture medium). In other words, the cell culture material can be composed of a porous carbon material which is produced from a plant-derived material having a silicon content of not less than 5 wt.% and which has a value of specific surface area determined by the nitrogen BET method of not less than 10 $m^2$/g, a silicon content of not more than 1 wt.%, and pore volumes determined by the BJH method and the MP methods of not less than 0.1 $cm^3$/g. To be more specific, powdery polylactic acid and the porous carbon material of Example 2-1 were mixed together, and the resulting mixture was molded, to obtain a sheet-shaped cell culture material composed of a thin film of 0.5 mm in thickness. By a method in which proteins and the like serving as growth factors for cells are adsorbed onto and sustainedly released from the porous carbon material constituting the cell culture material, it is possible to cultivate cells on the cell culture material easily and assuredly. Specifically, by a method in which cell growth factors necessary for cell culture (for instance, epidermal growth factor, insulin-like growth factor, transforming growth factor, nerve growth factor, etc.) are adsorbed onto and sustainedly released from the porous carbon material constituting the cell culture material, it is possible to culture various cells efficiently.

## Claims

1. Use of an adsorbent, comprising a porous carbon material which is produced from a plant-derived material having a silicon content of not less than 5 wt.% and which has a value of specific surface area determined by the nitrogen BET method of not less than 10 $m^2$/g, a silicon content of not more than 1 wt.%, and pore volumes determined by the BJH method and the MP method of not less than 0.1 $cm^3$/g, for adsorbing indole or uric acid or adenosine or α-amylase or 3-methylindole or tryptophan or indican or theophylline or inosine 5-monophosphate disodium salt or adenosine 5-tirphosphate disodium salt or a fatty acid or a coloring matter or hydrophobic molecules or for a renal disease drug or for a functional food.

## Patentansprüche

1. Verwendung eines Adsorbens, welches ein aus einem aus Pflanzen gewonnenen Material mit einem Siliciumgehalt von nicht weniger als 5 Gew.-% hergestelltes poröses Kohlenstoffmaterial mit einer gemäß der Stickstoff-BET-Methode bestimmten spezifischen Oberfläche von nicht weniger als 10 $m^2$/g, einen Siliciumgehalt von nicht mehr als 1 Gew.-% und gemäß der BJH-Methode und der MP-Methode bestimmte Porenvolumina von nicht weniger als 0,1 $cm^3$/g aufweist, umfasst, zum Adsorbieren von Indol oder Harnsäure oder Adenosin oder α-Amylase oder 3-Methylindol oder Tryptophan oder Indican oder Theophyllin oder Inosin-5-monophosphat-Dinatriumsalz oder Adenosin-5-triphosphat-Dinatriumsalz oder einer Fettsäure oder einem färbenden Material oder hydrophoben Molekülen oder für ein Arzneimittel zur Behandlung einer Nierenkrankheit oder für ein Functional Food.

## Revendications

1. Utilisation d'un adsorbant, comprenant un matériau de carbone poreux qui est produit à partir d'un matériau dérivé de plantes, ayant une teneur en silicium non inférieure à 5% en poids et qui possède une valeur de surface spécifique déterminée par la méthode BET d'azote non inférieure à 10 $m^2$/g, une teneur en silicium non supérieure à 1% en poids, et des volumes de pores déterminés par la méthode BJH et la méthode MP non inférieurs à 0,1 $cm^3$/g, pour l'adsorption d'indole ou d'acide urique ou d'adénosine ou d'a-amylase ou de 3-méthylindole ou de tryptophane ou d'indican ou de théophylline ou de sel disodique d'inosine-5'-monophosphate ou de sel disodique d'adénosine-5'-triphosphate ou d'un acide gras ou d'une matière colorante ou de molécules hydrophobes ou pour un médicament destiné à une maladie rénale ou pour un aliment fonctionnel.

# FIG.1

(A)

(B)

# FIG.2

# FIG.3

# F I G . 4

# F I G . 5

# FIG.6

# F I G . 7

(A)

(B)

# FIG.8

(A)

(B)
INGESTION AMOUNT (5-DAY AVERAGE (g))

# FIG.9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3565994 B
- JP 3719790 B
- WO 9627911 A
- JP SHO6211611 B
- JP 2002167325 A
- JP HEI9111296 B
- JP 2000053558 A
- EP 2060535 A1
- EP 2324854 A1
- EP 2330078 A1

**Non-patent literature cited in the description**

- **SCHETTINO M. A. et al.** *Quim. Nova,* 2007, vol. 30 (7), 1663-1668